# EUROPEAN PATENT APPLICATION

(11) **EP 3 975 195 A1**
(43) Date of publication of application: **30.03.2022**
(21) Application number: 20198849.0
(22) Date of filing: 29.09.2020
(51) Int. Cl.: G16H 30/40, G06T 7/10

(54) **METHOD, COMPUTER PROGRAM AND COMPUTER SYSTEM FOR USE IN THE PROCESSING OF MEDICAL IMAGES**

(71) Applicant: RaySearch Laboratories AB, 103 65 Stockholm (SE)
(72) Inventor: Ruffa, Giorgio, 112 63 Stockholm (SE); Trincavelli, Marco, 125 45 Älvsjö (SE); Gruselius, Hanna, 167 44 Bromma (SE)

(57) **Abstract**

Machine learning system that can be used for ensuring uniform and correct labeling of organs in segmentation of medical images, based on the properties of the organ and any label previously assigned to the organ in question, is disclosed.

## Description

### Technical Field

The present invention relates to a machine-learning system for use in planning a medical procedure and method of developing and using such a system.

### Background

When preparing for a medical procedure, the preparations are often based on one or more images of the patient. In many cases, for radiation therapy or surgery, the image needs to be contoured or segmented, that is, at least one of the structures in the image, should be delineated and identified. Using radiation therapy as an example, this will enable planning to take into account any region of the patient that should be exposed to a certain dose and any region that should be exposed as little as possible.

In the process of delineating, a label for each delineated structure is set, naming the structure. The name is normally chosen to inform about the type of structure, for example a tumor or a particular body part, such as the right kidney, a knee or the esophagus.

In recent years, machine learning systems are being used to an increasing extent in medical procedures. This requires the use of already available data to be used to train the machine learning model. For example, to identify a type of tumor, a number of images including this type of tumor should be input as training data, together with data informing about the surrounding structures. A lot of such data is already available to research institutions and clinics. To be useful as training data the machine learning system needs to be able to identify the structures based on the names given in the images. The names are often set by individuals processing the images, and may be very different. The task of manually renaming the structures in such data sets would be overwhelming and also prone to human error. A simple spell-check algorithm would also be prone to misinterpretation. Manually curated translation tables are difficult to handle and still error-prone.

It is an object of the invention to enable the use of existing image data for training machine learning systems. It is another object of the invention to facilitate the collaboration and sharing of data between different clinics. It is yet another object to enable script automation and analysis of available clinical data.

### Summary of the invention

The invention relates to a computer-based method of identifying one or more structures in a medical image, comprising the steps of
a. selecting an input image including at least one structure that is to be labelled,
b. identifying at least one feature of the structure,
c. in a machine-learning system trained to recognize the structure, classifying the structure based on the at least one feature,
d. in the machine-learning system, assigning a structure label to the structure, said structure label being selected from a set of approved labels.

This ensures that only approved labels are used for the structures, thus enabling the creation of a set of labels that can be interpreted by a machine, such as a machine learning system.

The method may further comprise the step of submitting any undecided structure to manual check, enabling a manual override if the machine learning system cannot make a decision.

In preferred embodiments, the method further comprises the steps of
e. identifying any previous label assigned to the structure,
f. obtaining a translation of the previous label to a translated label, said translated label being selected from the set of approved labels,
g. Comparing the translated label and the structure label and based on the result of the comparison, setting the actual label of the structure to the translated label or marking the structure as undecided.

Steps e and f are preferably performed in a machine learning system trained to translate an incorrect label assigned to a structure to a label from the set of approved labels. In this way, the system can be used to correct previous labels already assigned to structures, while taking into account the intention of the incorrect previous label.

The at least one feature may include one or more of the size and/or surface of the object, data related to the content of the object, and/or a value based on the size and the surface. The content of the object may be related to the values contained in the medical image in the area of the object.

The invention also relates to a method of training a machine learning system to assign a correct label to a structure in a medical image comprising the steps of submitting a set of training input data to the machine learning system, said training input data comprising a plurality of records, each record comprising at least a first structure representing the spatial location of a region of interest inside a patient and an approved label from a set of approved labels, matching the spatial location, and training the machine learning system to select the approved label matching the first structure based on its spatial location

Each record in the training input data may further comprise the information content of the medical image and the machine learning system may be trained to select the approved label matching the first structure based on its spatial location and the information content of the medical image.

The training method may preferably comprise the step of submitting a validation input data set, said validation input data comprising a plurality of records, each record comprising at least a first structure representing the spatial location of a region of interest inside a patient and a label from a set of approved labels, matching the spatial location to the machine learning system and tune at least one hyperparameter of the system based on the validation input data set.

The machine learning system may be tested by a method involving the step of submitting a test data set said test input data comprising a plurality of records, each record comprising at least a first structure representing the spatial location of a region of interest inside a patient and an approved label from a set of approved labels, matching the spatial location to the machine learning system to test the machine learning system.

The method may further comprise refining the model by combining the training data set, the validation data set and the test data set, and retraining the machine learning system applying the hyperparameters as previously tuned.

The invention also relates to a machine learning system trained according to the above, and the use of such a machine learning system to assign a correct approved label to a structure in a medical image.

Examples of queries that are enabled by the present method are to retrieve all right lung contours in the material, or to select all H-N cases.

The images used according to the invention may be 2D or 3D images.

### Brief description of drawings

The invention will be described in more detail in the following, by way of examples and with reference to the appended drawings.
Figure 1 is a flowchart of a general machine-learning based method of determining the correct label
Figure 2 illustrates the overall procedure according to the invention, including two different methods of ensuring standardized naming.
Figure 3 is a schematic representation of a comprehensive system for standardized labeling of organs at risk and for subsequent analysis of the standardized dataset.
Figures 4A and 4B illustrate an example of how a machine learning system may be trained.

### Detailed description of embodiments

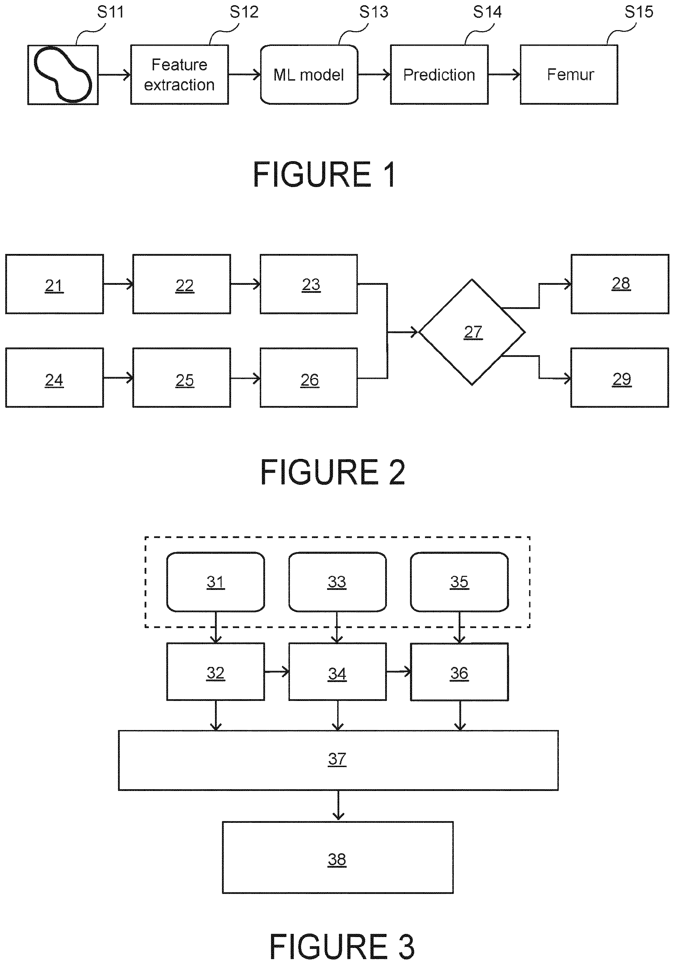

Figure 1 is an overall flowchart of a method that may be used according to the present invention. An image S 11 including a delineated structure, for example, an organ at risk, is subjected to feature extraction in step S 12 and the extracted information is input to a machine learning system S 13 that has been trained according to one of the procedures that will be discussed below. The machine learning system S13 outputs a classification score S14 indicative of the likelihood that the structure in question belongs to a particular class, each class corresponding to a particular type of organ or structure, such as "heart", or "right kidney". The system then, in step S15, selects the appropriate label for the structure that has been identified, according to a standardized list of unique labels for the different structures, used during training.

The encoding in step S 12, also called feature extraction, means calculating a set of features for a given data point. In general, machine learning models make predictions on a series of so-called "features" of the original data. In the present case, a feature can be, for example, the volume, or the area of the surface, of the contoured regions. A feature can also be more complex or a mathematical composition of several features, such as the volume/surface ratio or a function related to image moments, that is, a certain particular weighted average (moment) of the image pixels' intensities. This may be achieved in two different ways.

The features may be calculated either manually or automatically, both of which are known in the art. With manual feature engineering, an expert on machine learning has selected and engineered a series of features. With automatic feature engineering, some algorithms, notably deep learning algorithms, are able to automatically develop relevant features during the training phase. Some of these features might be interpretable by a human, although in most cases they are not.

Figure 2 illustrates two separate ways of standardizing the naming of structures in existing medical images according to the invention represented by an uppermost chain S21 - S23 for interpretation of existing labels and a lowermost chain S24 - S26 for generating labels based on image data. Input data to the lowermost chain S24 - S26 include one or more delineated images and a set of standard names to be used in the labels. For the uppermost chain, S21 - S23, the existing label is used as input data. In a preferred embodiment the two methods are used together, as will be discussed below. The images may be in two or three dimensions.

The procedure illustrated by the uppermost chain S21 - S23 is based on interpretation of the original label S21 which has been assigned earlier and which typically does not adhere to the standard name to be used for the structure. The label is first translated to common language in a step S22, so that, for example, the labels opticn_L, optic_NRV_L, Left_OpticNerve, etc, will all be interpreted as denoting the left optic nerve. In a subsequent step S23, the approved name for the left optic nerve is obtained from the set of approved standard names and is output.

Any suitable machine translation algorithm may be used for the translation, including rule-based systems, translation tables, or machine learning based machine translation. The system should be able to recognize all possible names that are used in the available images for different structures that are used in existing segmentations and match them to the names that should be used according to the set of standard names. In other words, as an example, the machine learning system must be trained to identify, based on each of the labels opticn:L, optic_NRV_L, Left_OpticNerve, that the structure is the left optic nerve and to assign the correct label according to the standardized set, for example, optic_nerve_left. Similarly, LungR, lung right, RightLung, etc., should all be interpreted as identifying the right lung, and assigned an approved label reflecting this, for example, lung right. For training a machine learning system for this purpose, training sets of assigned names with associated standardized names for each of them should be used. As is common in the art, the context of the term may also be considered, typically in the form of the n-most closest words to the term.

The procedure illustrated by the lowermost chain S24 - S26 is based on analysis of the image data S24. The geometric features of the image are extracted in a step 25. The geometric features may include features of the ROI itself, such as its size and shape. The geometric features may also include features of surrounding structures or tissue, so that the ROI can be identified not only based on its own features but additionally or alternatively on which structures it is close to. Based on the geometric features, the ROI is identified and the approved label is obtained in step S26 from the set of standard names, and is output.

The output from one of the steps S23 and S26 may be used to label the ROI without using any of the other steps shown in Figure 2. To further eliminate the risk of error, they are preferably used together. In this case, both outputs are submitted to a comparison unit S27. If the result of the comparison is that both outputs are the same, the label is assumed to be correct and may be used as it is as indicated in step S28. If the comparison shows that the outputs are different, the structure is marked to be checked again, preferably by manual check as in step S29, to resolve the conflict between the two machine-based labels in steps S23 and S26. In this way, only the structures that cannot be unambiguously named by the machine-based procedures need to be handled manually, reducing the manual workload involved in renaming the structures.

The uppermost method of Figure 2 can be implemented by machine learning methods, or by other methods. In the first case, to train a machine-learning system for use in the uppermost method of Figure 2, a set of all possible names that are used in the available images for different structures that are used in existing segmentations should be matched to the names that should be used according to the set of standard names. In other words, the machine learning system must be trained to identify, based on the labels opticn_L, optic_NRV_L, Left OpticNerve, the left optic nerve and to assign the correct label, for example optic_nerve_left. Similarly, labels such as LungR, Lung_right, RightLung, etc., should all be interpreted as identifying the right lung, and assigned a uniform label reflecting this from the set of standardized names, for example lung_right.

To train a machine-learning system for use in the lowermost method of Figure 2, sets of training data must be provided to the machine learning system. The training sets include ROI contours and approved standardized labels for each structure. Preferably, but not necessarily, the training sets also include the outer contour of the respective patient and/or one or more medical images of the patient. The medical images may be of any suitable modality, CT, MRI etc.

Figure 3 is an overall illustration of a system that may be used to assist data standardization, for consistent labeling of organs at risk, and subsequent analysis of the standardized dataset trough an analytic platform, including the structure shown separately in Figure 2. In the upper left portion of the Figure is the ROI block 31 handling binary ROI data, corresponding to the step 24 in Figure 2. This data is provided to a feature extraction block 32, corresponding to the step S25 in Figure 2. The system also includes a label module 33 holding original label data, corresponding to step S21 in Figure 2. The label data from 33 are provided to an OAR standardization block 34, corresponding to the steps S22, S23, S26, S27, S28 and S29 in Figure 2. The output from the feature extraction block 32 is also provided to the standardization block 34. The system also includes a metadata block 35 holding treatment metadata arranged to provide such metadata to a disease site inference block 36, which is also arranged to receive data from the standardization block 34 and use those data to determine the body site, that is, the portion of the body that will receive the treatment. Depending on the location of the tumor this may be, for example, the pelvic area, or the thorax. The body site will normally be a subset of the total field of view of the image.

The outputs from the feature extraction block 32, the standardization block 34 and the disease site inference block 36 are provided to an analytics module 37 arranged to analyze the data for various purposes. Importantly, the analytics module 37 is arranged to perform the comparison in step S27 to decide on the correct label for each structure. The correct labels from the comparison performed in step S27 may be used by a number of possible applications that need a standardized dataset as input. Such applications are schematically represented in Figure 3 by a block 38 and may include, for example, one or more of the following:
- Label Autocorrection: one application may be arranged to suggest the standardized name to apply, while the specialist is delineating an organ. In this way the name will be preemptively corrected and won't even enter the system in a not-standardized form
- Insights: valuable information about the patient population. This usually involves finding unknown patterns in the data and confirming known ones.
- Outlier Detection: finding patients or cases that are outside of the main distribution. This may involve, for example, finding out that a particular set of pelvic cases had an abnormally high dosage. Or that a particular patient received a very high dosage in the organs at risk because he/she was already treated before.
- Patient Selection: selecting a set of patient/cases to perform any kind of clinical study and/or to train a machine learning model. This application strongly interacts with the outlier detection part. In many cases, outliers should be excluded from a study because they are not representative, while in other cases, the focus should be solely on the outliers.
- Quality assurance (QA): an umbrella term for all the operations that concerns comparing a plan to a population of other plans and see how well it meets acceptance criteria to be delivered.

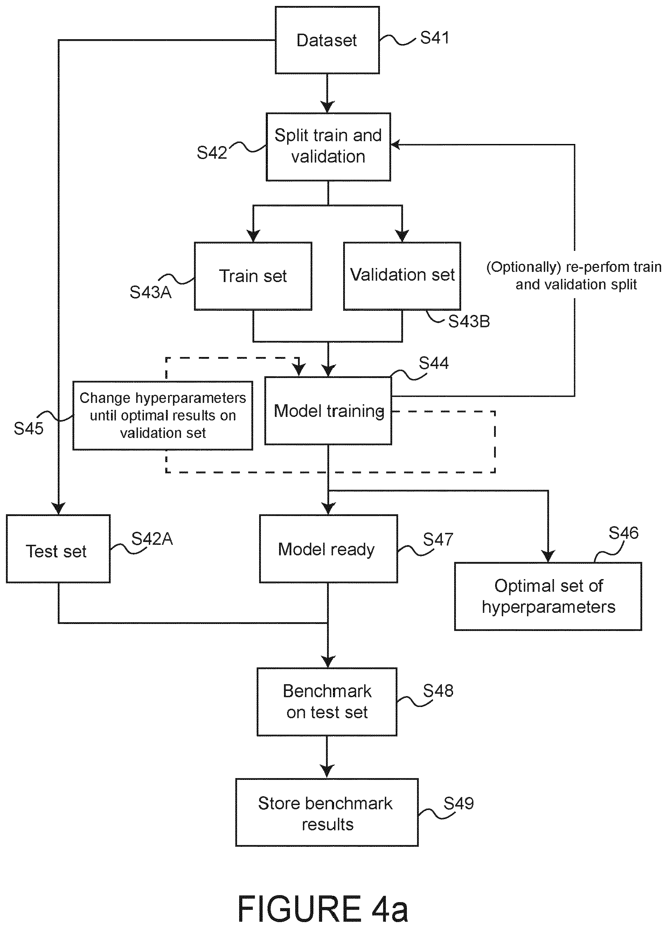

Figure 4A illustrates how a machine learning system may be trained for use according to the present invention. In a first step S41 a total data set to be used for training is provided. The total data set comprises a number of records, each record comprising one or more of 3D or 2D structures representing the spatial location of a region of interest inside the patient and a standardized label for that region of interest, taken from a set of approved labels. Optionally, each record also comprises information on the content of the medical image containing the 3D or 2D structure. The machine learning system also has access to the set of approved labels.

In step S42, a test data set 42A comprising a subset of the records in the total data set to be used for testing the machine learning system after training is split out from the total data set. Next, in step S43 the remaining records in the total data set are divided into a training data set 43A that is used to train the model and a validation data set 43B that will be used to fine tune certain hyperparameters of the machine learning system after training. it should be noted that the training data set and the validation data set may be amended with respect to both content and size during the training process, whereas the test data set is normally fixed throughout the process.

In step S44, the model is trained, using the training set, to train the machine learning system to match the structure to the correct label from the set of approved labels. In an optional step S45, the trained model is fine-tuned using the validation set to determine the optimal set of hyperparameters. This may be done manually, automatically or semi-automatically and is conventionally done by adjusting the hyperparameters until optimal results are achieved based on the validation set. Algorithms for optimizing the hyperparameters are known in the art. The optimal set of hyperparameters is output from the model training step S44/S45, in step S46, to be used in the machine learning system. The final model is output from the model training step S44/S45 in step S47. In a subsequent step S48, the test set 42A is submitted to the machine learning system to test and benchmark the trained system with the optimal set of hyperparameters. In step S49, the benchmark results are stored.

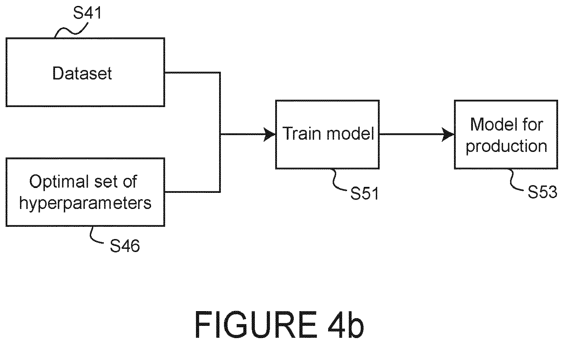

Before using the model in production, the following additional steps may be performed as shown in Figure 4B: The total data set from step S41 and the optimal set of hyperparameters S46 may be input to the trained system and used to train S51 the system once more. The outcome of this procedure is an even more reliable system S53 for selecting the appropriate labels for the regions of interest.

## Claims

1. A computer-based method of identifying at least one structure in a medical image, comprising the steps of
a. selecting an input image including at least one structure that is to be labelled,
b. identifying at least one feature of the structure,
c. in a machine-learning system trained to recognize the structure, classifying the structure based on the at least one feature,
d. in the machine-learning system, assigning a structure label to the structure, said structure label being selected from a set of approved labels.

2. A computer-based method according to claim 1, further comprising the step of submitting any undecided structure to manual check.

3. A method according to claim 1 or 2, further comprising the steps of
e. identifying any previous label assigned to the structure,
f. obtaining a translation of the previous label to a translated label, said translated label being selected from the set of approved labels,
g. Comparing the translated label and the structure label and based on the result of the comparison, setting an approved label of the structure to the translated label or marking the structure as undecided.

4. A method according to claim 3, wherein steps e and f are performed in a machine learning system trained to translate an incorrect previous label assigned to a structure to an approved label from the set of approved labels.

5. A method according to any one of the preceding claims, wherein the at least one feature includes the size and/or surface of the object, data related to the content of the object, and/or a value based on the size and the surface.

6. A method of training a machine learning system to assign a correct label to a structure in a medical image comprising the steps of submitting a set of training input data to the machine learning system,
said training input data comprising a plurality of records, each record comprising at least a first structure representing the spatial location of a region of interest inside a patient and an approved label from a set of approved labels, matching the spatial location,
training the machine learning system to select the label matching the first structure based on its spatial location

7. A method according to claim 6, wherein each record in the training input data further comprises the information content of the medical image and the machine learning system is trained to select the approved label matching the first structure based on its spatial location and the information content of the medical image.

8. A method according to claim 6 or 7, comprising the step of submitting a validation input data set, said validation input data comprising a plurality of records, each record comprising at least a first structure representing the spatial location of a region of interest inside a patient and a label from a set of approved labels, matching the spatial location to the machine learning system and tune at least one hyperparameter of the system based on the validation input data set.

9. A method according to any one of the claims 6 - 8, comprising the step of submitting a test data set said test input data comprising a plurality of records, each record comprising at least a first structure representing the spatial location of a region of interest inside a patient and a label from a set of approved labels, matching the spatial location to the machine learning system to test the machine learning system.

10. A method according to claim 9, comprising the step of combining the training data set, the validation data set and the test data set, and retrain the machine learning system applying the hyperparameters as tuned in claim 8.

11. A machine learning system trained according to any one of the claims 6 - 10.

12. Use of a machine learning system trained according to any one of the claims 6 - 10, to assign an approved label to a structure in a medical image.
